# EUROPEAN PATENT APPLICATION

(11) **EP 1 295 609 A1**
(43) Date of publication of application: **26.03.2003**
(21) Application number: 01906193.6
(22) Date of filing: 22.02.2001
(51) Int. Cl.: A61K 45/06, A61K 31/44, A61K 31/405, A61K 31/198, A61K 31/702, A61K 31/133, A61K 31/70, A61P 3/10, A61P 43/00

(54) **DRUGS CONTAINING COMBINED ACTIVE INGREDIENTS**

(30) Priority: 24.02.2000 JP 2000052297
(71) Applicant: Takeda Chemical Industries, Ltd., Osaka-shi, Osaka 541-0045 (JP)
(72) Inventor: SUGIYAMA, Yasuo, Kawanishi-shi, Hyogo 666-0111 (JP); ODAKA, Hiroyuki, Kobe-shi, Hyogo 651-1223 (JP); SAKIYAMA, Hiroshi, Nagoya-shi, Aichi 468-0069 (JP); IWASAKI, Masato, Tokyo 112-0002 (JP); FUNATSU, Masami, Neyagawa-shi, Osaka 572-0019 (JP)
(74) Representative: Lewin, John Harvey
(86) International application number: JP0101282
(87) International publication number: WO01062295

(57) **Abstract**

A pharmaceutical agent comprising only an α-glucosidase inhibitor in combination with a non-sulfonylurea insulinsecretagogue as active ingredients is useful as an agent for the prophylaxis or treatment of diabetes and the like.

## Description

### Technical Field

The present invention relates to a pharmaceutical agent comprising only an α-glucosidase inhibitor in combination with a non-sulfonylurea insulin secretagogue as active ingredients, which is useful as an agent for the prophylaxis or treatment of diabetes and the like.

### Background Art

α-Glucosidase inhibitors are known for a combined use with, for example, sulfonylurea insulin secretagogues, and such combined use is described in, for example, the following publications.
1) Takeda Kenkyujo ho (J. Takeda Res. Lab.), vol. 54, pp. 21-33 (1995) describes a combined use of voglibose, an α-glucosidase inhibitor, and sulfonylurea.
2) Yakuri to Chiryo (Japanese Pharmacology and Therapeutics), vol. 22, No. 9, pp. 3759-3770 (1994) describes a combined use of voglibose and glibenclamide or gliclazide, which is sulfonylurea.
3) J. Int. Med. Res., vol. 24, No. 5, pp. 433-437 (1996) describes a combined use of an α-glucosidase inhibitor and tolubutamide, which is a sulfonylurea insulin secretagogue.
4) Ann. Pharmacother., vol. 30, No. 11, pp. 1255-1262 (1996) describes a combined use of acarbose, which is an α-glucosidase inhibitor, and sulfonylurea.
5) Sogo-Rinsho (Clinic All-round), vol. 45, No. 12, pp. 2760-2764 (1996) describes a combined use of an α-glucosidase inhibitor such as acarbose, voglibose and the like, and sulfonylurea.

In addition, WO99/03478 describes a combined use of an α-glucosidase inhibitor, an insulin sensitizer and an insulin secretagogue.

However, none of the above-mentioned publications describe a combined use only of an α-glucosidase inhibitor and a non-sulfonylurea insulin secretagogue as active ingredients.

Thus, there is a demand for a combination drug having properties sufficiently superior as a pharmaceutical agent, such as a superior prophylactic and/or therapeutic effect on diabetes and the like without side effects, and the like.

### Disclosure of the Invention

The present invention relates to:
(1) a pharmaceutical agent comprising only an α-glucosidase inhibitor in combination with a non-sulfonylurea insulin secretagogue as active ingredients;
(2) the pharmaceutical agent of the aforementioned (1), wherein the α-glucosidase inhibitor is voglibose;
(3) the pharmaceutical agent of the aforementioned (1), wherein the α-glucosidase inhibitor is acarbose;
(4) the pharmaceutical agent of the aforementioned (1), wherein the α-glucosidase inhibitor is miglitol;
(5) the pharmaceutical agent of the aforementioned (1), wherein the α-glucosidase inhibitor is emiglitate;
(6) the pharmaceutical agent of the aforementioned (1), wherein the non-sulfonylurea insulin secretagogue is repaglinide, nateglinide or mitiglinide;
(7) the pharmaceutical agent of the aforementioned (1), wherein the non-sulfonylurea insulin secretagogue is repaglinide;
(8) the pharmaceutical agent of the aforementioned (1), wherein the non-sulfonylurea insulin secretagogue is nateglinide;
(9) the pharmaceutical agent of the aforementioned (1), wherein the non-sulfonylurea insulin secretagogue is mitiglinide;
(10) the pharmaceutical agent of the aforementioned (1), wherein the α-glucosidase inhibitor is voglibose, and the non-sulfonylurea insulin secretagogue is repaglinide;
(11) the pharmaceutical agent of the aforementioned (1), which is an agent for the prophylaxis or treatment of diabetes;
(12) the pharmaceutical agent of the aforementioned (1), which is an agent for the prophylaxis or treatment of a diabetic complication;
(13) the pharmaceutical agent of the aforementioned (1), which is an agent for the prophylaxis or treatment of impaired glucose tolerance;
(14) a method for treating diabetes, which comprises administering effective amounts of only an α-glucosidase inhibitor and a non-sulfonylurea insulin secretagogue as active ingredients to a mammal;
(15) use of an α-glucosidase inhibitor for the production of an agent for the prophylaxis or treatment of diabetes, which is used in combination only with a non-sulfonylurea insulin secretagogue as an active ingredient;
(16) a method for reducing side effects of active ingredients, which comprises administering effective amounts of only an α-glucosidase inhibitor and a non-sulfonylurea insulin secretagogue as said active ingredients to a diabetic patient;
(17) a method for reducing doses of active ingredients, which comprises administering effective amounts of only an α-glucosidase inhibitor and a non-sulfonylurea insulin secretagogue as said active ingredients to a diabetic patient; and the like.

The "α-glucosidase inhibitor" to be used in the present invention need only be a drug that inhibits digestive enzymes such as amylase, maltase, α-dextrinase, sucrase and the like and delays the digestion of starch and sucrose. As the "α-glucosidase inhibitor", for example, voglibose, acarbose, miglitol, emiglitate and the like are mentioned. Of these, voglibose is preferable.

In the present invention, two or more α-glucosidase inhibitors may be used in combination at a suitable ratio.

The "non-sulfonylurea insulin secretagogue" to be used in the present invention need only be a drug having a promoting action on the secretion of insulin from pancreatic B cells and free of a sulfonylurea skeleton. As the "non-sulfonylurea insulin secretagogue", for example, repaglinide, nateglinide, mitiglinide (KAD-1229), GLP (glucagon-like peptide)-1, GLP-1(7-36)-amide, V8-GLP-1 (LY-307161), praqmlintide (AC-137), exendin-4 (AC-2993), DPP-728-A, glymidine, glybuzole, V-411, JT-608 and the like are mentioned. The "non-sulfonylurea insulin secretagogue" is preferably repaglinide, nateglinide or mitiglinide. Of these, repaglinide is preferable. These non-sulfonylurea insulin secretagogues are rapid releasing type or ultra-rapid releasing type drugs, which show a promoting action on the secretion of insulin from pancreatic B cells in a short time after administration.

In the present invention, two or more non-sulfonylurea insulin secretagogues may be used in combination at a suitable ratio.

In the pharmaceutical agent of the present invention, a preferable combination is exemplified by:
1) combination of voglibose and repaglinide;
2) combination of voglibose and nateglinide;
3) combination of voglibose and mitiglinide;
4) combination of acarbose and repaglinide;
5) combination of acarbose and nateglinide;
6) combination of acarbose and mitiglinide;
7) combination of miglitol and repaglinide;
8) combination of miglitol and nateglinide;
9) combination of miglitol and mitiglinide; and the like.

Of these, 1) combination of voglibose and repaglinide; 2) combination of voglibose and nateglinide; and 3) combination of voglibose and mitiglinide are preferable, and the combination of voglibose and repaglinide is particularly preferable.

The pharmaceutical agent of the present invention can be obtained by combining an α-glucosidase inhibitor and a non-sulfonylurea insulin secretagogue, that are active ingredients. These active ingredients may be formulated by mixing separately or simultaneously with a pharmacologically acceptable carrier according to a method known *per se* [conventional method in the technical field of manufacturing pharmaceutical preparations, such as the method described in the Japanese Pharmacopoeia (e.g., JP XIII)].

The dosage form of the pharmaceutical agent or each active ingredient of the present invention is, for example, an oral preparation such as tablet, capsule (including soft capsule and microcapsule), powder, granule, syrup and the like; and a parenteral preparation such as injection (e.g., subcutaneous injection, intravenous injection, intramuscular injection, intraperitoneal injection etc.), external preparation (e.g., preparation for nasal administration, percutaneous preparation, ointment etc.), suppository (e.g., rectal suppository, vaginal suppository etc.), pellet, drops, sustained-release preparation (e.g., sustained-release microcapsule etc.) and the like.

The production methods of oral preparations and parenteral preparations are explained in detail in the following.

An oral preparation can be produced by adding, to the active ingredients, for example, an excipient (e.g., lactose, saccharose, starch, D-mannitol, xylitol, sorbitol, erythritol, microcrystalline cellulose, light silicic anhydride etc.), a disintegrant (e.g., calcium carbonate, starch, carboxymethyl cellulose, carboxymethyl cellulose calcium, low-substituted hydroxypropylcellulose, croscarmellose sodium, carboxymethyl starch sodium, light silicic anhydride etc.), a binder (e.g., pregelatinized starch, gum arabic, carboxymethyl cellulose, hydroxypropylcellulose, hydroxypropylmethylcellulose, polyvinylpyrrolidone, microcrystalline cellulose, methyl cellulose, saccharose, D-mannitol, trehalose, dextrin etc.), a lubricant (e.g., talc, magnesium stearate, calcium stearate, colloidal silica, polyethylene glycol 6000 etc.) and the like, and compression-molding the mixture. To the oral preparation, an acid such as hydrochloric acid, phosphoric acid, malonic acid, succinic acid, DL-malic acid, tartaric acid, maleic acid, fumaric acid, citric acid and the like or a base such as sodium carbonate, sodium hydrogencarbonate, sodium citrate, sodium tartrate and the like may be added for promoting dissolution of the active ingredients.

Furthermore, the oral preparation may be coated by a method known per se for the purpose of masking a taste, enteric coating, or achieving a sustained release. Examples of the coating agent include an enteric polymer (e.g., cellulose acetate phthalate, methacrylic acid copolymer L, methacrylic acid copolymer LD, methacrylic acid copolymer S, hydroxypropylmethylcellulose phthalate, hydroxypropylmethylcellulose acetate succinate, carboxymethylethylcellulose etc.), a gastric coating polymer (e.g., polyvinylacetal diethylaminoacetate, aminoalkyl methacrylate copolymer E etc.), a water-soluble polymer (e.g., hydroxypropylcellulose, hydroxypropylmethylcellulose etc.), a water-insoluble polymer (e.g., ethylcellulose, aminoalkyl methacrylate copolymer RS, ethyl acrylate/methyl methacrylate copolymer etc.), wax and the like. For coating, a plasticizer such as polyethylene glycol etc. and a shading agent such as titanium oxide, iron sesquioxide etc. may be used, along with the above-mentioned coating agents.

An injection can be produced by dissolving, suspending or emulsifying an active ingredient in an aqueous solvent (e.g., distilled water, physiological saline, Ringer's solution etc.), an oily solvent (e.g., vegetable oil such as olive oil, sesame oil, cottonseed oil, corn oil etc.; propylene glycol, macrogol, tricaprylin etc.) and the like, together with a dispersant [e.g., Tween 80 (Atlas Powder Co., U.S.A.), HCO 60 (Nikko Chemicals Co., Ltd.), polyethylene glycol, carboxymethyl cellulose, sodium alginate etc.], a preservative (e.g., methyl paraben, propyl paraben, benzyl alcohol, chlorobutanol, phenol etc.), an isotonizing agent (e.g., sodium chloride, glycerine, D-sorbitol, D-mannitol, xylitol, glucose, fructose etc.) and the like.

Where desired, additives may be used, such as a dissolution aid (e.g., sodium salicylate, sodium acetate, polyethylene glycol, propylene glycol, D-mannitol, trehalose, benzyl benzoate, ethanol, Tris aminomethane, cholesterol, triethanolamine, sodium carbonate, sodium citrate etc.), a suspending agent (e.g., surfactant such as stearyltriethanolamine, sodium lauryl sulfate, laurylaminopropionic acid, lecithin, benzalkonium chloride, benzethonium chloride, glycerine monostearate etc.); a hydrophilic polymer such as polyvinyl alcohol, polyvinylpyrrolidone, carboxymethylcellulose sodium, methylcellulose, hydroxymethylcellulose, hydroxyethylcellulose, hydroxypropylcellulose etc.), a buffering agent (e.g., buffer of phosphate, acetate, carbonate, citrate etc.), a stabilizer (e.g., human serum albumin etc.), a soothing agent (e.g., propylene glycol, lidocaine hydrochloride, benzyl alcohol etc.), a preservative (e.g., p-oxybenzoic acid esters, chlorobutanol, benzalkonium chloride, benzyl alcohol, phenethyl alcohol, dehydroacetic acid, sorbic acid etc.), and the like.

An external preparation can be produced by forming an active ingredient into a solid, semisolid or liquid composition. For example, the above-mentioned solid composition can be produced by making a powder of an active ingredient as it is or upon addition of and mixing with an excipient (e.g., lactose, D-mannitol, starch, crystalline cellulose, saccharose etc.), a thickening agent (e.g., natural gum, cellulose derivative, acrylate polymer etc.) and the like. The above-mentioned liquid composition can be produced in almost the same manner as in the case of injections. A semisolid composition is preferably an aqueous or oily gel, or an ointment. These compositions may contain a pH adjuster (e.g., phosphoric acid, citric acid, hydrochloric acid, sodium hydroxide etc.), a preservative (e.g., p-oxybenzoic acid esters, chlorobutanol, benzalkonium chloride, benzyl alcohol, phenethyl alcohol, dehydroacetic acid, sorbic acid etc.), and the like.

A suppository is produced by forming an active ingredient into an oily or aqueous solid, semisolid or liquid composition. As an oily base to be used for production of the composition may be, for example, higher fatty acid glyceride [e.g., cocoa butter, witepsols (Huels Aktiengesellschaft, Germany) etc.], a medium chain fatty acid triglyceride [e.g., Migriols (Huels Aktiengesellschaft, Germany) etc.], a vegetable oil (e.g., sesame oil, soybean oil, cottonseed oil etc.), and the like. As an aqueous base, for example, polyethylene glycols, propylene glycol and the like are mentioned. As an aqueous gel base, for example, natural gum, cellulose derivative, vinyl polymer, acrylate polymer and the like are mentioned.

The mode of administration of the pharmaceutical agent of the present invention is not particularly limited, as long as an α-glucosidase inhibitor and a non-sulfonylurea insulin secretagogue are combined on administration. Examples of such administration mode include (1) administration of a single preparation obtained by simultaneous formulation of the α-glucosidase inhibitor and the non-sulfonylurea insulin secretagogue, (2) simultaneous administration by the same administration route of two kinds of preparations obtained by separate formulation of the α-glucosidase inhibitor and the non-sulfonylurea insulin secretagogue, (3) staggered administration by the same administration route of two kinds of preparations obtained by separate formulation of the α-glucosidase inhibitor and the non-sulfonylurea insulin secretagogue, (4) simultaneous administration by different administration routes of two kinds of preparations obtained by separate formulation of the α-glucosidase inhibitor and the non-sulfonylurea insulin secretagogue, (5) staggered administration by different administration routes of two kinds of preparations obtained by separate formulation of the α-glucosidase inhibitor and the non-sulfonylurea insulin secretagogue, such as administration in the order of the α-glucosidase inhibitor and then the non-sulfonylurea insulin secretagogue, or in the reversed order, and the like. Of these, the above-mentioned (2) and (3) are preferable.

More specifically, the α-glucosidase inhibitor and the non-sulfonylurea insulin secretagogue are separately formed into oral preparations such as tablet and the like, and the oral preparations are preferably administered simultaneously or in a time staggered manner.

In the pharmaceutical agent of the present invention, the α-glucosidase inhibitor and the non-sulfonylurea insulin secretagogue are preferably administered simultaneously or in a staggered manner before meal (e.g., 5 - 60 min, preferably 15 - 30 min, before meal).

The pharmaceutical agent of the present invention shows low toxicity and can be administered safely to a mammal (e.g., human, mouse, rat, rabbit, dog, cat, cattle, horse, pig, monkey etc.) orally or parenterally.

The dose of the pharmaceutical agent of the present invention follows the dose of each drug and can be appropriately determined depending on the administration subject, age and body weight of the administration subject, symptom, administration time, dosage form, administration method, combination of drugs and the like.

The doses of the α-glucosidase inhibitor and the non-sulfonylurea insulin secretagogue follow a clinical dose and can be appropriately determined based thereon.

For example, when α-glucosidase inhibitor is administered to an adult diabetic patient (body weight 50 kg), the daily dose is generally 0.01 - 1000 mg, preferably 0.1 - 500 mg, which dose can be administered once or several times a day in divided portions.

Particularly, when voglibose is used as the α-glucosidase inhibitor, the daily dose is generally 0.1 - 2 mg, preferably 0.5 - 1 mg. It is particularly preferred that 0.2 - 0.3 mg, preferably 0.2 mg, of voglibose be orally administered 3 times a day before meal.

When a non-sulfonylurea insulin secretagogue is administered to an adult diabetic patient (body weight 50 kg), the daily dose is generally 0.01 - 1000 mg, preferably 0.1 - 500 mg, which dose can be administered once or several times a day in divided portions.

The following describes in detail the dose of a non-sulfonylurea insulin secretagogue when administered to an adult diabetic patient (body weight 50 kg).

The daily dose of repaglinide is generally 0.1 - 20 mg, preferably 0.5 - 15 mg. It is particularly preferred that 0.5 - 4 mg, preferably 2 mg, of repaglinide be orally administered 3 times a day before meal.

The daily dose of nateglinide is generally 10 - 1000 mg, preferably 50 - 600 mg. It is particularly preferred that 50 - 200 mg, preferably 90 mg, of nateglinide be orally administered 3 times a day before meal.

The daily dose of mitiglinide is generally 0.5 - 100 mg, preferably 1 - 50 mg. It is particularly preferred that 1 - 10 mg, preferably 5 mg, of mitiglinide be orally administered 3 times a day before meal.

The daily dose of GLP-1 or GLP-1(7-36)-amide is generally 0.1 - 10 mg, preferably 0.5 - 5 mg. It is particularly preferred that 0.1 - 1 mg, preferably 0.4 mg, of GLP-1 or GLP-1(7-36)-amide be orally administered 3 times a day before meal as a buccal.

The daily dose of V8-GLP-1 is generally 0.1 - 50 mg, preferably 0.2 - 20 mg. V8-GLP-1 is particularly preferably administered as a buccal, an oral agent and a subcutaneous or intramuscular preparation including a sustained-release preparation.

The daily dose of praqmlintide is generally 10 - 1000 µg, preferably 50 - 500 µg. It is particularly preferred that 30 - 100 µg of praqmlintide be subcutaneously or intravenously administered 2 or 3 times a day.

The daily dose of exendin is generally 0.1 - 500 µg, preferably 0.2 - 100 µg. It is particularly preferred that 0.5 - 50 µg of exendin-4 be subcutaneously administered.

The daily dose of DPP-728-A is generally 1 - 2000 mg, preferably 5 - 1000 mg. It is particularly preferred that 10 - 500 mg of DPP-728-A be orally administered.

The daily dose of glymidine is generally 0.1 - 500 mg, preferably 0.5 - 100 mg. It is particularly preferred that 1 - 50 mg of glymidine be orally administered.

The daily dose of glybuzole is generally 0.1 - 500 mg, preferably 0.5 - 100 mg. It is particularly preferred that 1 - 50 mg of glybuzole be orally administered.

The daily dose of V-411 is generally 1 mg - 10 g, preferably 5 mg - 5 g. It is particularly preferred that 20 mg - 1 g of V-411 be orally administered.

In the pharmaceutical agent of the present invention, the mixing ratio of an α-glucosidase inhibitor and a non-sulfonylurea insulin secretagogue can be appropriately determined depending on the administration subject, age and body weight of the administration subject, symptom, administration time, dosage form, administration method, combination of drugs and the like. For example, the non-sulfonylurea insulin secretagogue is used in an amount of generally about 0.0001 - 100 parts by weight, preferably about 0.001-10 parts by weight, per part by weight of the α-glucosidase inhibitor.

The pharmaceutical agent and each active ingredient of the present invention can be used as a prophylactic or therapeutic agent of diabetes (e.g., type 1 diabetes, type 2 diabetes, gestational diabetes etc.); a prophylactic or therapeutic agent of impaired glucose tolerance (IGT); an agent for suppressing development of impaired glucose tolerance into diabetes; a prophylactic or therapeutic agent of hyperlipidemia (e.g., hypertriglyceridemia, hypercholesterolemia, hypo-high-density-lipoproteinemia etc.); a prophylactic or therapeutic agent of hyperinsulinemia; a prophylactic or therapeutic agent of diabetic complications (e.g., retinopathy, nephropathy, neuropathy, macroangiopathy etc.); a prophylactic or therapeutic agent of coronary and cerebrovascular disorders; a prophylactic or therapeutic agent of hyperammonemia; a prophylactic or therapeutic agent of obesity; a prophylactic or therapeutic agent of hypertension, visceral obesity and insulin resistance that cause syndrome X; a prophylactic or therapeutic agent of bone metabolism disorders such as osteopenia, osteoporosis and the like; an agent for controlling appetite; a prophylactic or therapeutic agent of gastrointestinal diseases such as fatty liver, hepatitis, constipation, diarrhea, enteritis and the like; a prophylactic or therapeutic agent of dumping syndrome; a prophylactic or therapeutic agent of glycogenosis; intestinal flora normalization agent based on lactic acid bacteria-increasing action; and the like.

For diagnostic criteria of diabetes, the Japan Diabetes Society reported new diagnostic criteria in 1999.

According to this report, diabetes is a condition showing any of a fasting blood glucose level (glucose concentration in venous plasma) of not less than 126 mg/dl, a 2 h value (glucose concentration in venous plasma) of a 75 g oral glucose tolerance test (75 g OGTT) of not less than 200 mg/dl, and a non-fasting blood glucose level (glucose concentration in venous plasma) of not less than 200 mg/dl. A condition that does not fall under the above-mentioned diabetes and "a condition showing a fasting blood glucose level (glucose concentration in venous plasma) of less than 110 mg/dl and a 2 h value (glucose concentration in venous plasma) of a 75 g oral glucose tolerance test (75 g OGTT) of less than 140 mg/dl" (normal type) is called a "borderline type".

In addition, new diagnostic criteria of diabetes were reported by ADA (American Diabetes Association) in 1997 and by WHO in 1998.

According to these reports, too, diabetes is a condition showing a fasting blood glucose level (glucose concentration in venous plasma) of not less than 126 mg/dl or a 2 h value (glucose concentration in venous plasma) of a 75 g oral glucose tolerance test of not less than 200 mg/dl.

According to the above-mentioned reports, impaired glucose tolerance is a condition showing a 2 h value (glucose concentration in venous plasma) of a 75 g oral glucose tolerance test of not less than 140 mg/dl and less than 200 mg/dl. According to the report of ADA, a condition showing a fasting blood glucose level (glucose concentration in venous plasma) of not less than 110 mg/dl and less than 126 mg/dl is called IFG (Impaired Fasting Glucose). According to the report of WHO, among the IFG (Impaired Fasting Glucose), only a condition showing a 2 h value (glucose concentration in venous plasma) of a 75 g oral glucose tolerance test of less than 140 mg/dl is called IFG (Impaired Fasting Glycemia).

The pharmaceutical agent of the present invention can be also used as a prophylactic or therapeutic agent of diabetes, borderline type, impaired glucose tolerance, IFG (Impaired Fasting Glucose) and IFG (Impaired Fasting Glycemia), as determined according to the above-mentioned new diagnostic criteria. Moreover, the pharmaceutical agent of the present invention can prevent the progress of borderline type, impaired glucose tolerance, IFG (Impaired Fasting Glucose) or IFG (Impaired Fasting Glycemia) into diabetes.

The diabetes treatment effect (e.g., hypoglycemic action etc.) possessed by the pharmaceutical agent of the present invention can be evaluated by, for example, measuring venous plasma glucose concentration or Hb (hemoglobin) A1c of the subject of administration before and after administration of said pharmaceutical agent, and comparing the obtained concentrations between before and after the administration. As used herein, by HbA1c is meant glycosylated hemoglobin, which is gradually produced in response to the blood glucose concentration. Thus, HbA1c is valued as a blood glucose control index that resists influence of rapid changes in the blood glucose level of diabetic patients. The pharmaceutical agent of the present invention has a superior HbA1c-lowering effect.

When a secondary action (e.g., gastrointestinal disorders such as diarrhea etc. and the like) is caused by each active ingredient of the present invention or a combined administration thereof, such action can be reduced by a combined use of the pharmaceutical agent or each active ingredient of the present invention with a dietary fiber.

In addition, the secondary action (e.g., gastrointestinal disorders such as diarrhea etc., insulin hypersecretion and the like) that each active ingredient has can be reduced by gradually increasing the dose from a level lower than usual to a higher dose of the pharmaceutical agent and each active ingredient of the present invention.

Moreover, the pharmaceutical agent and each active ingredient of the present invention afford a sufficient effect at a dose lower than usual by administering in admixture with meals.

The pharmaceutical agent of the present invention shows superior pharmaceutical properties [e.g., enhanced hypoglycemic action, reduceed amount of active ingredients used, reduced secondary action (e.g., gastrointestinal disorders such as diarrhea etc., insulin hypersecretion and the like), improved stability, improved bioavailability, improved internal absorption and the like], as compared to a single use of each active ingredient.

In particular, the pharmaceutical agent of the present invention affords suppression of insulin hypersecretion, which is caused by a non-sulfonylurea insulin secretagogue, by an α-glucosidase inhibitor. Thus, the pharmaceutical agent of the present invention is extremely useful as a prophylactic or therapeutic agent of diabetes, which is associated with a less risk of causing various diseases (e.g., vascular complications, hypoglycemia, arteriosclerosis, obesity and the like) due to insulin hypersecretion.

### Best Mode for Embodying the Invention

The present invention is described in more detail in the following by means of Reference Examples and Example, which are not to be construed as limitative.

### Reference Example 1

According to a conventional method, a tablet having the following composition is produced.

| (Composition per 1 tablet) | |
|---|---|
| 1) voglibose | 0.3 mg |
| 2) cornstarch | 35.0 mg |
| 3) hydroxypropylcellulose | 5.8 mg |
| 4) lactose | 0.6 mg |
| 5) magnesium stearate | q.s. |
| total | 200.0 mg |

### Reference Example 2

According to a conventional method, a tablet having the following composition is produced.

| (Composition per 1 tablet) | |
|---|---|
| 1) voglibose | 0.2 mg |
| 2) cornstarch | 23.0 mg |
| 3) hydroxypropylcellulose | 0.4 mg |
| 4) lactose | 0.6 mg |
| 5) magnesium stearate | q.s. |
| total | 130.0 mg |

### Example 1

The hypoglycemic effect of the pharmaceutical agent of the present invention can be evaluated by conducting the following experiments to a single diabetic patient.
1) Voglibose (0.2 mg tablet) and repaglinide (2 mg tablet) are administered to a diabetic patient before breakfast, and the blood glucose level is measured at 1 hour after the meal.
2) In the same manner as in the above-mentioned 1) except the use of a placebo tablet instead of the voglibose 0.2 mg tablet, the blood glucose level of the diabetic patient is measured at 1 hour after the meal.
3) In the same manner as in the above-mentioned 1) except the use of a placebo tablet instead of the repaglinide 2 mg tablet, the blood glucose level of the diabetic patient is measured at 1 hour after the meal.

The blood glucose level measured in the above-mentioned 1) shows a significant decrease as compared to the blood glucose levels measured in the above-mentioned 2) and 3).

### Example 2

An effect of the combined use of the α-glucosidase inhibitor (voglibose) and non-sulfonylurea insulin secretagogue (nateglinide) was examined in an oral sucrose loading test using Sprague-Dawley rats (SD rats).

SD rats (7-week-old, male) were divided into 6 groups (6 rats per group) to give 1) a control group (distilled water administration), 2) a voglibose 0.1 mg/kg administration group, 3) a nateglinide 10 or 30 mg/kg administration group, and 4) a voglibose 0.1 mg/kg and nateglinide 10 or 30 mg/kg combined administration group. After fasting overnight, every rat was given the above-mentioned drugs by single or combined oral administration, and successively given 2.5 g/kg sucrose solution by oral administration. The blood was drawn from the rat tail vein before administration of the sucrose solution and 15, 30, 60 and 120 min after the administration, and the plasma glucose level was measured. The plasma glucose level was measured by an enzymatic method using an L type Wako Glu2 (Wako Pure Chemical Industries, Ltd.).

Increase in the plasma glucose area was calculated by a trapezoid method (Odaka et al., J. Nutr. Sci. Vitaminol., vol. 38, p. 37 (1992) using the plasma glucose level at each time point. The results are shown in Table 1.

**Table 1**

| Drug (dose:mg/kg) | Increase in plasma glucose area (mg· h/dl) |
|---|---|
| control | 86±25 |
| voglibose (0.1) | 30±10** |
| nateglinide (10) | 65±28 |
| nateglinide (30) | 54±15* |
| voglibose (0.1) + nateglinide (10) | 14± 8**##& |
| voglibose (0.1) + nateglinide (30) | 7±13**##&& |

| | |
|---|---|
| average ± standard deviation *:p<0.05, | |
| **:p<0.01 (Dunnett's test relative to control) | |
| ##:p<0.01 (T test relative to nateglinide 10 or 30 mg/kg group) | |
| &:p<0.05, | |
| &&: p<0.01 (T test relative to voglibose 0.1 mg/kg group) | |

As shown in Table 1, the combined use of voglibose and nateglinide significantly reduced increase in the plasma glucose area as compared to the administration of each drug alone. In other words, the combined use of the both drugs afforded a superior hypoglycemic effect.

### Example 3

An effect of the combined use of the α-glucosidase inhibitor (voglibose) and the non-sulfonylurea insulin secretagogue (repaglinide) was examined in an oral sucrose loading test using Sprague-Dawley rats (SD rats).

SD rats (10-week-old, male) were divided into 4 groups (6 rats per group) to give 1) a control group (distilled water administration), 2) a repaglinide 0.05 mg/kg administration group, and 3) a voglibose 0.1 mg/kg and repaglinide 0.05 mg/kg combined administration group. After fasting overnight, every rat was given the above-mentioned drugs by single or combined oral administration, and successively given 2.5 g/kg of a sucrose solution by oral administration. The blood was drawn from the rat tail vein before administration of the sucrose solution and 15, 30 and 60 min after the administration, and the plasma glucose level was measured. The plasma glucose level was measured by an enzymatic method using an L type Wako Glu2 (Wako Pure Chemical Industries, Ltd.), and the plasma insulin level was measured by radio immunoassay using Insulotech-Mochida (Mochida Pharmaceutical Co., Ltd.).

Increase in the plasma glucose and plasma insulin areas was calculated by a trapezoid method (Odaka et al., J. Nutr. Sci. Vitaminol., vol. 38, p. 37 (1992)) using the plasma glucose and plasma insulin levels at each time point. The results are shown in Table 2.

**Table 2**

| Drug (dose:mg/kg) | Increase in plasma glucose area (mg·h/dl) | Increase in plasma insulin area (µU·h/ml) |
|---|---|---|
| control | 38±9 | 213±83 |
| repaglinide(0.05) | 6±13** | 432±117 |
| repaglinide(0.05) + voglibose (0.1) | -8±8** | 121±42# |

| | | |
|---|---|---|
| average ± standard deviation **:p<0.01 (Dunnett's test relative to control) | | |
| #:p<0.05 (T test relative to repaglinide 0.05 mg/kg group) | | |

As shown in Table 2, the combined use of voglibose and repaglinide significantly lowered the blood glucose level while reducing the insulin hypresecretion observed in the single administration of repaglinide. In other words, the combined use of the both drugs afforded a superior hypoglycemic effect.

### Industrial Applicability

The pharmaceutical agent of the present invention shows superior pharmaceutical properties [e.g., enhanced hypoglycemic action, reduced amount of active ingredients used, reduced secondary action (e.g., gastrointestinal disorders such as diarrhea etc., insulin hypersecretion, hypoglycemia etc.), improved stability, improved bioavailability, improved internal absorption and the like], as compared to a single use of each active ingredient.

## Claims

1. A pharmaceutical agent comprising only an α-glucosidase inhibitor in combination with a non-sulfonylurea insulin secretagogue as active ingredients.

2. The pharmaceutical agent of claim 1, wherein the α-glucosidase inhibitor is voglibose.

3. The pharmaceutical agent of claim 1, wherein the α-glucosidase inhibitor is acarbose.

4. The pharmaceutical agent of claim 1, wherein the α-glucosidase inhibitor is miglitol.

5. The pharmaceutical agent of claim 1, wherein the α-glucosidase inhibitor is emiglitate.

6. The pharmaceutical agent of claim 1, wherein the non-sulfonylurea insulin secretagogue is repaglinide, nateglinide or mitiglinide.

7. The pharmaceutical agent of claim 1, wherein the non-sulfonylurea insulin secretagogue is repaglinide.

8. The pharmaceutical agent of claim 1, wherein the non-sulfonylurea insulin secretagogue is nateglinide.

9. The pharmaceutical agent of claim 1, wherein the non-sulfonylurea insulin secretagogue is mitiglinide.

10. The pharmaceutical agent of claim 1, wherein the α-glucosidase inhibitor is voglibose, and the non-sulfonylurea insulin secretagogue is repaglinide.

11. The pharmaceutical agent of claim 1, which is an agent for the prophylaxis or treatment of diabetes.

12. The pharmaceutical agent of claim 1, which is an agent for the prophylaxis or treatment of diabetic complication.

13. The pharmaceutical agent of claim 1, which is an agent for the prophylaxis or treatment of impaired glucose tolerance.

14. A method for treating diabetes, which comprises administering effective amounts of only an α-glucosidase inhibitor and a non-sulfonylurea insulin secretagogue as active ingredients to a mammal.

15. Use of an α-glucosidase inhibitor for the production of an agent for the prophylaxis or treatment of diabetes, which is used in combination only with a non-sulfonylurea insulin secretagogue as an active ingredient.

16. A method for reducing the side effects of active ingredients, which comprises administering effective amounts of only an α-glucosidase inhibitor and a non-sulfonylurea insulin secretagogue as said active ingredients to a diabetic patient.

17. A method for reducing doses of active ingredients, which comprises administering effective amounts of only an α-glucosidase inhibitor and a non-sulfonylurea insulin secretagogue as said active ingredients to a diabetic patient.
